# EUROPEAN PATENT SPECIFICATION

(11) **EP 3 445 314 B1**
(45) Date of publication and mention of the grant of the patent: **22.12.2021**
(21) Application number: 17713963.1
(22) Date of filing: 28.03.2017
(51) Int. Cl.: A61K 8/24, A61K 8/25, A61K 8/36, A61Q 11/00, A61K 8/73, A61K 8/81, A61P 1/02

(54) **ORAL CARE COMPOSITION**
MUNDPFLEGEZUSAMMENSETZUNG
COMPOSITION DE PROTECTION ORALE

(30) Priority: 18.04.2016 EP 16165739
(43) Date of publication of application: 27.02.2019
(73) Proprietor: Unilever IP Holdings B.V., 3013 AL Rotterdam (NL); Unilever Global IP Limited, Wirral, CH62 AZD (GB)
(72) Inventor: BLASCO, Alessandro, 26841 Casalpusterlengo (LO) (IT); LANDI, Giovanna, Neuchatel 2000 (CH)
(74) Representative: Tansley, Sally Elizabeth
(86) International application number: PCT/EP2017/057336
(87) International publication number: WO 2017/182240

(56) References cited:
- EP-A1- 0 346 957
- WO-A1-00/48561
- WO-A1-2014/184083
- WO-A2-2007/076444
- WO-A2-2010/054494
- WO-A2-2010/090855
- WO-A2-2014/184084
- DATABASE GNPD [Online] MINTEL; February 2016 (2016-02), "Fluoride Toothpaste", XP002760018, Database accession no. 3751097

## Description

### Field of the Invention

The present invention relates to compositions for treating dental hypersensitivity.

### Background and Prior Art

Dental hypersensitivity is a painful condition affecting up to 20% of the adult population. There are two categories of therapy for the treatment of tooth hypersensitivity based upon two modes of action. The first category, nerve-depolarising agents, are pharmaceutical agents such as potassium nitrate, which function by interfering with neural transduction of the pain stimulus.

The second category, known as occluding agents, function by physically blocking the exposed ends of the dentinal tubules, thereby reducing dentinal fluid movement and reducing the irritation associated with the shear stress described by the hydrodynamic theory.

An example of an occluding agent is found in US 5,270,031 which describes a tubule occluding desensitizer comprising a polyacrylic acid such as Carbopol^{®} polymeric materials. Another tubule occluding composition is disclosed in US 5,374,417 which discloses a potassium salt of a synthetic anionic polymer, such as a polycarboxylate.

There is a continuing need for effective compositions to which mitigate the effects of hypersensitivity of the teeth.

### Description of the Invention

The present invention relates an oral composition comprising a sensitivity mitigating agent comprising a mixture of hydrated silica, a potassium citrate, and calcium hydroxyapatite and a polymeric deposition aid, comprising a polymer based on a copolymer of methyl vinyl ether and maleic anhydride and a polymeric thickening agent comprising xanthan gum, the composition having a viscosity 3 weeks from manufacture or longer of from 10 to 60 Pa.s (10,000-60,000 cP) at 25°C (measured using a Brookfield viscometer with Helipath Stand and a T-bar, TB spindle, rotating at 5 rpm)

### Detailed Description of the Invention

Compositions of the invention have a viscosity 3 weeks from manufacture or longer of from 10 to 60 Pa.s (10,000-60,000 cP) at 25°C. Preferably, the viscosity 3 weeks from manufacture is from 15 to 45 Pa.s (15,000-45,000 cP) at 25°C, more preferably from 19 to 35 Pa.s (19,000-35,000 cP) at 25°C.

In the context of the present invention viscosity is measured at 25°C, using a Brookfield viscometer with Helipath Stand and a T-bar, TB spindle, rotating at 5 rpm. The measurement jar has a minimum diameter of 5 cm and a minimum height of 10 cm. The viscosity value is recorded 60 seconds from the start of the measurement.

Compositions according to the invention will usually contain an aqueous continuous phase. The amount of water generally ranges from 40 to 89% by weight based on the total weight of the composition, more preferably from 50 to 70 wt%.

Preferably, compositions according to the invention will further comprise humectants. The amount of humectant generally ranges from 10 to 50%, more preferably from 15 to 40% by weight based on the total weight of the composition. Preferred humectants that are present at the above levels include polyols, particularly preferred are sorbitol, glycerol and mixtures thereof.

The compositions comprise a xanthan gum,. It is particularly preferred if both xanthan gum and gellan gum are used. It is preferred if the level of xanthan gum is from 0.15 to 1.2 wt% of the total composition, more preferably from 0.4 to 0.9 wt%. It is preferred if the level of gellan gum is from 0.01 to 2 wt% , more preferably from 0.02 to 1 wt% of the composition. If used in combination it is preferred if the total level of Xanthan and gellan gum is from 0.5 to 0.9 wt% of the composition.

It is preferable if the weight ratio of Xanthan gum to gellan gum is from 2:1 to 20:1.

The composition comprises ingredients that mitigate tooth sensitivity, a preferred ingredient for this effect is hydrated silica. Preferably the hydrated silica has a mean particle size lower than 15µm, more preferably lower than 10, most preferably from 1 to 6 µm. It is preferable if the hydrated silica described above is present at levels from 1 to 10 wt% of the total composition, more preferably from 3 to 8 wt% of the total composition.

Compositions of the invention comprise potassium citrate salt, It is preferable if the levels of potassium citrate salt is from is from 1 to 10 wt% of the total composition, more preferably from 2 to 5 wt% of the total composition. Such salts help prevent dental sensitivity.

Compositions according to the invention comprise the dental sensitivity agent HAP (Calcium Hydroxyapatite). Preferably the level of HAP is from 0.5 to 10 wt% of the total composition, more preferably from 1 to 6 wt% of the total composition.

It is preferable if the total level of anti-sensitivity agents is from 5 to 15 wt% of the total composition.

Preferably, the composition comprises at least two sensitivity mitigating agents, more preferably at least three anti sensitivity agents. It is especially preferred if the anti-sensitivity agents comprise calcium hydroxyapatite, hydrated silica and potassium citrate.

It is preferable if the weight ratio of calcium hydroxyapatitie to hydrated silica is from 1:1 to 1:4; and/or the weight ratio of potassium citrate to hydrated silica is from 1:1 to 1:4 and/or the weight ratio of potassium citrate to calcium hydroxyapatite is from 2:1 to 1:2.

It is particularly beneficial if the weight ratio of the total weight of anti-sensitivity agent to deposition agent is from 5:1 to 30:1.

Compositions of the invention comprise a zinc salt, preferably zinc sulphate, zinc citrate or zinc chloride, more preferably zinc sulphate. Preferably the level of zinc salt is from 0.05 to 1.0 wt% of the total formulation more preferably from 0.1 to 0.5 wt%.

It is preferable if the level of ethanol in the total composition is less than 0.1 wt%.

Compositions of the invention may comprise a preservative. A preferred preservative is sodium benzoate, more preferably a system formed from ethylhexylglycerin, phenoxyethanol and benzyl alcohol. Preferably the level of preservative in the case of the mixed system described is from 0.5 to 2 wt% of the total composition. This level refers to the total level of preservative system.

It is preferable if the pH of the composition at 25° C is from 5.6 to 8.0, more preferably from 6.0 to 7.5.

The composition of the invention may comprise a deposition aid. The term "deposition aid" in the context of this invention generally means a material which aids deposition of agents that mitigate tooth sensitivity from the composition.

Use of the composition in the context of this invention typically involves application of the composition to the teeth by use of an applicator.

Suitable deposition aids for use in the invention will generally dissolve or disperse in water at a temperature of 25°C.

Preferred deposition aids for use in the invention are water-soluble. The term "water-soluble" in this particular context generally means that the deposition aid has an aqueous solubility of at least 10g/L at 25°C, and more preferably at least 30g/L at 25°C (where the solubility is determined in un-buffered distilled water). It is particularly preferable that the deposition aid remains water-soluble after drying, so that it can be re-dissolved. This prevents undesirable build-up of the deposition aid on the teeth after repeated usage of the composition.

Suitable deposition aids for use in the invention include polymeric materials, preferably polymeric materials which are water soluble as defined above.

Polymeric materials for use as deposition aids in the invention may be naturally or synthetically-derived, and may be ionic or nonionic in nature.

Preferably such polymeric materials are high molecular weight. The term "high molecular weight" in this particular context generally means that the polymeric material has a molecular weight of at least 50,000, more preferably at least 500,000 g/mol. A suitable method to determine the molecular weight of such polymeric materials is gel permeation chromatography against a polyethylene glycol standard.

Specific examples of suitable classes of polymeric material for use as deposition aids in the invention include:

Water-soluble, high molecular weight linear homopolymers of ethylene oxide characterised by the general formula H(OCH₂CH₂)ₙOH. These materials are generally termed polyethyleneoxides (or altematively, polyoxyethylenes, or polyethylene glycols). In the general formula, n usually has an average value of at least 2000, preferably at least 50,000.

Water-soluble, high molecular weight cellulose ethers such as methylcellulose, hydroxyethylcellulose, hydroxypropylcellulose, hydroxypropyl methylcellulose, hydroxybutyl methylcellulose, hydroxyethyl ethylcellulose, sodium carboxymethylcellulose, and sodium carboxymethyl hydroxyethylcellulose.

A preferred class of polymeric material for use as deposition aids in the invention includes water-soluble, high molecular weight polymers having anionic side groups along the polymer main chain.

Specific examples of such materials include poly(carboxylic acid) polymers. Poly (carboxylic acid) polymers are typically polymers which include -COOH groups in their structure, or groups which are derived from -COOH groups such as salt, ester or anhydride groups.

For example, the poly(carboxylic acid) polymers may include:

-[C(R¹)(COOH)-]-

units in their structure, in which R¹ is selected from hydrogen, C₁₋₃ alkyl, C₁₋₃ alkoxy or C₁₋₃ hydroxyalkyl. Preferably R¹ is hydrogen.

A preferred type of poly (carboxylic acid) polymer includes adjacent:

-[C(R¹)(COOH)-]-

units in its structure (where R¹ is as defined above), for example polymers based on maleic acid, which typically include:

―{-CH(COOH)-CH(COOH)-]-

units, and/or salts or esters of such units, or such units in anhydride form in which -COOH groups on adjacent carbon atoms are cyclised to form a ring system.

For example, the poly(carboxylic acid) polymers may comprise units with pairs of carboxylic acid groups on adjacent polymer chain carbon atoms, for example polymers comprising:

-[-C(R¹)(R²)-C(R³)(R⁴)-C(R⁵)(COOH)- C(R⁶)(COOH)-]-

units in its structure (and/or salts or esters of such units, or such units in anhydride form in which - COOH groups on adjacent carbon atoms are cyclised to form a ring system); in which R¹,R²,R³,R⁴,R⁵ and R⁶ are each independently selected from hydrogen, C₁₋₃ alkyl or C₁₋₃ alkoxy. Preferably R¹ and R² are hydrogen, R³ is hydrogen and R⁴ is methoxy and R⁵ and R⁶ are hydrogen.

Compositions of the invention comprise a polymeric deposition aid based on a copolymer of methyl vinyl ether and maleic anhydride, and is commercially available for example under the trade name Gantrez^{®}.

A particularly preferred example of such a polymer comprises:

-[-CH₂-CH(OCH₃)-CH(COOH)-CH(COOH)-]-

units in its structure, in which the -COOH groups are in free acid form. Such polymers may be linear or cross-linked. More preferably the polymer is linear. Polymers of this type are commercially available for example under the trade name Gantrez^{®} S. Most preferably such a polymer has a molecular weight of at least 500,000 g/mol (e.g. Gantrez^{®} S-96), ideally at least 1,000,000 g/mol (e.g. Gantrez^{®} S-97).

Alternative polymers which may be used comprise the units as described above in anhydride form, i.e. in which the two adjacent -COOH groups are cyclised to form a ring system. Such polymers are commercially available under the tradename Gantrez^{®} AN, e.g. Gantrez^{®} AN-119, Gantrez^{®} AN-903, Gantrez^{®} AN-139 and Gantrez^{®} AN-169.

Other alternative polymers which may be used comprise the units as described above in partial salt form, for example in which some of the free -COOH groups are converted into a metal salt of a Group I or Group II metal such as either sodium or calcium, or a mixed sodium-calcium salt. Such polymers are commercially available under the tradename Gantrez^{®} MS, e.g. Gantrez^{®} MS-955.

Other alternative polymers which may be used comprise the units as described above in partial ester form, for example in which some of the free ―COOH groups are esterified with C₁₋₆ alkyl, e.g. ethyl or n-butyl. Such polymers are commercially available under the tradename Gantrez^{®} ES, e.g. Gantrez^{®} ES-225 or Gantrez^{®} ES-425.

Mixtures of any of the above described materials may also be used.

The amount of deposition aid (as defined above) in compositions of the invention suitably ranges from 0.001 to 5.0%, preferably from 0.005 to 4.0wt%, more preferably from 0.01 to 2.0wt% by total weight deposition aid (as defined above) based on the total weight of the composition.

The composition may contain low levels of surfactant based on the total weight of the composition. If present, the surfactant is preferably present at levels of less than 3 wt% of the total composition, more preferably at levels from 0.2 to 2 wt%, most preferably at levels of less from 0.5 to 1.5 wt% of the total composition.

Suitable surfactants include anionic surfactants, such as the sodium, magnesium, ammonium or ethanolamine salts of C₈ to C₁₈ alkyl sulphates (for example sodium lauryl sulphate), C₈ to C₁₈ alkyl sulphosuccinates (for example dioctyl sodium sulphosuccinate), C₈ to C₁₈ alkyl sulphoacetates (such as sodium lauryl sulphoacetate), C₈ to C₁₈ alkyl sarcosinates (such as sodium lauryl sarcosinate), C₈ to C₁₈ alkyl phosphates (which can optionally comprise up to 10 ethylene oxide and/or propylene oxide units) and sulphated monoglycerides.

Other suitable surfactants include nonionic surfactants, such as optionally polyethoxylated fatty acid sorbitan esters, ethoxylated fatty acids, esters of polyethylene glycol, ethers of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used. Steareth 30 an ether of polyethylene glycol is particularly preferred.

Compositions of the present invention may also contain further optional ingredients customary in the art such as fluoride ion sources, anticalculus agents, buffers, flavouring agents, sweetening agents, colouring agents, opacifying agents, further antisensitivity agents and antimicrobial agents.

The invention will now be illustrated by the following non-limiting Examples. Examples of the invention will be illustrated by a number, comparative Examples by a letter.

### Examples

Composition was prepared according to Table 1

**Table 1**

| **Ingredient** | **Wt%** | | |
|---|---|---|---|
| | **Example A** | **Example B** | **Example 1** |
| Water and minors | To 100 | To 100 | To 100 |
| Sorbitol | 15.0 | 15.0 | 15.0 |
| Glycerine | 15.0 | 15.0 | 15.0 |
| Hydrated Silica | 5.0 | 5.0 | 5.0 |
| Potassium Citrate | 3.0 | 3.0 | 3.0 |
| Calcium Hydroxyapatite | 2.0 | 2.0 | 2.0 |
| Steareth-30 | 1.0 | 1.0 | 1.0 |
| Xanthan Gum | 0.1 | 1.5 | 0.75 |
| Methyl vinyl ether and maleic acid copolymer | 0.5 | 0.5 | 0.5 |
| Zinc Sulphate | 0.20 | 0.20 | 0.20 |
| Sodium Monofluorophosphate | 0.17 | 0.17 | 0.17 |
| Gellan Gum | 0.05 | 0.05 | 0.05 |
| Viscosity (cps)* | 6,560 | 111,000 | 21,680 |

| | | | |
|---|---|---|---|
| * 3 weeks after manufacturing. Where 1 cps=1 cP= 10⁻³ Pa.s | | | |

The following experiments demonstrate the ability of the compositions of the invention to adhere to the surface of human teeth.

### Experiment 1

10 dried human teeth were weighed and then rehydrated for 10 mins into sterilized human saliva before being weighed again. The teeth were immersed in Example A, pulled out and weighed for a 3^{rd} time.

The teeth were suspended (crown facing down, roots facing up), into a container full of human saliva at 37°C -with gentle shaking to simulate saliva flow on teeth- for 1 minute. The teeth were removed from the container and support, and weighed for a 4^{th} time.

The differences between the differing weights were calculated and the final results expressed as % of product lost from the teeth, due to the flow of saliva.

The teeth were fully cleaned, dried, and the same process was repeated for Example 1.

Table 2 states the percentage of product lost after immersion for 1 minute in human saliva under gentle shaking at 37°C as described above.

**Table 2**

| **Example** | **% Product lost** |
|---|---|
| **Example 1** | **70.5** |
| **Example A** | **95.5*** |

| | |
|---|---|
| **asterisk indicates statistically significant difference (p<0.05) between test products. Statystical analysis was performed through t Student test, using SAS GMP 11 software.* | |

### Experiment 2

10 dried human teeth were weighed, soaked for 2 hours into sterilized human saliva at 37°C and weighed again.

The teeth were immersed into Example A, pulled out and weighed for a 3^{rd} time.

The teeth were left suspended (crowns facing down) at room temperature and weighed after 3 and 10 minutes for a 4^{th} and 5^{th} time. The differences between the weights were calculated and the results expressed as % of product lost (i.e. fallen off the teeth) after 3 and 10 minutes of suspension. The teeth were fully cleaned, dried and the above process repeated for Example 1.

Table 3 states the percentage product lost from tooth surface after 3 and 10 minutes

**Table 3**

| **Example** | **% Product lost 3 mins** | **% Product lost 10 mins** |
|---|---|---|
| **Example 1** | **12.89** | **25.68** |
| **Example A** | **43.88*** | **60.33**** |

| | | |
|---|---|---|
| **asterisk indicates statistically significant difference (p<0.05) between test products after 3 minutes.* ***two asterisks indicate statistically significant difference (p<0.05) between test products after 10 minutes.* | | |

*Statystical analysis was performed through t Student test, using SAS GMP 11 software.*

It is thus demonstrated that the Example of the invention adheres to the teeth better than the comparative Example.

### Experiment 3

12 grams of either Example B or Example 2 were put on the upper top of a previously scaled ceramic tile to form a uniform line. The tile was then fixed on a 45° inclined plane and pictures were taken at 15 seconds, 1, 4, 8, 15 30 and 60 minutes. The results were obtained by direct comparison of the pictures of the 2 test formulations, and further expressed as area covered in cm² per set amount of time (software used for the calculations: Image-Pro Plus).

**Table 4**

| **Product** | **Time in seconds** | | | | | | |
|---|---|---|---|---|---|---|---|
| | **15** | **60** | **240** | **480** | **900** | **1800** | **3600** |
| Example 1 | 32,44 cm² | 51,11 cm² | 64,09 cm² | 70,97 cm² | 76,52 cm² | 75,22 cm² | 78,40 cm² |
| Example B | 1,03 cm² | 1,64 cm² | 3,56 cm² | 5,40 cm² | 10,53 cm² | 19,88 cm² | 23,93 cm² |
| Difference | 31,41 cm² | 49,47 cm² | 60,53 cm² | 65,57 cm² | 65,99 cm² | 55,34 cm² | 54,47 cm² |

Table 4 demonstrates that Example 1 covered/coated a tile surface wider by more than 54 cm², compared to Example B.

The above Experiments demonstrated that the Example of the invention adheres to the teeth well and is easy to cover the teeth. It thus has improved anti-sensitivity properties as it remains on the teeth longer than the comparative Examples and is easier to apply.

## Claims

1. An oral care composition comprising a sensitivity mitigating agent comprising a mixture of hydrated silica, a potassium citrate, and calcium hydroxyapatite and a polymeric deposition aid, comprising a polymer based on a copolymer of methyl vinyl ether and maleic anhydride and a polymeric thickening agent comprising xanthan gum, the composition having a viscosity 3 weeks from manufacture or longer of from 10 to 60 Pa.s (10,000-60,000 cP) at 25°C (measured using a Brookfield viscometer with Helipath Stand and a T-bar, TB spindle, rotating at 5 rpm).

2. An oral care composition according to claim 1 in which the composition having a viscosity 3 weeks from manufacture or longer of from 15 to 45 Pa.s (15,000-45,000 cP) at 25°C.

3. An oral care composition according to claim 1 or claim 2 in which the polymeric deposition agent comprises a water soluble polymer and/or copolymer having anionic side groups along the polymer main chain.

4. An oral care composition according to any preceding claim in which the level of deposition agent is from 0.005 to 4wt% of the total composition.

5. An oral care composition according to any preceding claim in which the hydrated silica has a mean particle size from 1 to 6 µm.

6. An oral care composition according to any preceding claim in which the level of hydrated silica in the total composition is from 1 to 10 wt% of the total composition.

7. An oral care composition according to any preceding claim in which the level of potassium citrate is from 1 to 10 wt% of the total composition.

8. An oral care composition according to any preceding claim in which the level of calcium hydroxyapatite is from 1 to 8 wt% of the total composition.

9. An oral care composition according to any preceding claim in which the weight ratio of calcium hydroxyapatitie to hydrated silica is from 1:1 to 1:4.

10. An oral care composition according to any preceding claim in which the weight ratio of potassium citrate to hydrated silica is from 1:1 to 1:4.

11. An oral care composition according to any preceding claim in which the weight ratio of potassium citrate to calcium hydroxyl apatite is from 2:1 to 1:2.

12. An oral care composition according to any preceding claim in which the weight ratio of the total weight of anti-sensitivity agent to deposition agent is from 5:1 to 30:1.

13. An oral care composition according to any preceding claim in which the total level of anti-sensitivity agents is from 5 to 15 wt% of the total composition.

14. An oral care composition according to any preceding claim in which the composition further comprises gellan gum.

15. An oral care composition according to any preceding claim for use in the treatment of dental hypersensitivity.

## Patentansprüche

1. Mundpflegezusammensetzung, umfassend ein Mittel zur Milderung der Empfindlichkeit, umfassend eine Mischung von Kieselsäuregel, ein Kaliumcitrat und Calciumhydroxyapatit, und ein polymeres Abscheidungshilfsmittel, umfassend ein Polymer auf Basis eines Copolymers von Methylvinylether und Maleinsäureanhydrid, und ein polymeres Verdickungsmittel, umfassend Xanthangummi, wobei die Zusammensetzung 3 Wochen nach der Herstellung oder später eine Viskosität von 10.000 bis 60.000 cP bei 25 °C (gemessen unter Verwendung eines Brookfield-Viskosimeters mit Helipath-Stativ und einer T-Stange TB-Spindel, die mit 5 U/min rotiert) aufweist.

2. Mundpflegezusammensetzung nach Anspruch 1, bei der die Zusammensetzung 3 Wochen nach der Herstellung oder später eine Viskosität von 15.000 bis 45.000 cP bei 25 °C aufweist.

3. Mundpflegezusammensetzung nach Anspruch 1 oder Anspruch 2, bei der das polymere Abscheidungsmittel ein wasserlösliches Polymer und/oder Copolymer mit anionischen Seitengruppen entlang der Polymerhauptkette umfasst.

4. Mundpflegezusammensetzung nach irgendeinem vorhergehenden Anspruch, bei der der Gehalt an Abscheidungsmittel 0,005 bis 4 Gew.-% der gesamten Zusammensetzung beträgt.

5. Mundpflegezusammensetzung nach irgendeinem vorhergehenden Anspruch, bei der das Kieselsäuregel eine mittlere Teilchengröße von 1 bis 6 µm aufweist.

6. Mundpflegezusammensetzung nach irgendeinem vorhergehenden Anspruch, bei der der Gehalt an Kieselsäuregel in der gesamten Zusammensetzung 1 bis 10 Gew.-% der gesamten Zusammensetzung beträgt.

7. Mundpflegezusammensetzung nach irgendeinem vorhergehenden Anspruch, bei der der Gehalt an Kaliumcitrat 1 bis 10 Gew.-% der gesamten Zusammensetzung beträgt.

8. Mundpflegezusammensetzung nach irgendeinem vorhergehenden Anspruch, bei der der Gehalt an Calciumhydroxyapatit 1 bis 8 Gew.-% der gesamten Zusammensetzung beträgt.

9. Mundpflegezusammensetzung nach irgendeinem vorhergehenden Anspruch, bei der das Gewichtsverhältnis von Calciumhydroxyapatit zu Kieselsäuregel 1:1 bis 1:4 beträgt.

10. Mundpflegezusammensetzung nach irgendeinem vorhergehenden Anspruch, bei der das Gewichtsverhältnis von Kaliumcitrat zu Kieselsäuregel 1:1 bis 1:4 beträgt.

11. Mundpflegezusammensetzung nach irgendeinem vorhergehenden Anspruch, bei der das Gewichtsverhältnis von Kaliumcitrat zu Calciumhydroxylapatit 2:1 bis 1:2 beträgt.

12. Mundpflegezusammensetzung nach irgendeinem vorhergehenden Anspruch, bei der das Gewichtsverhältnis des Gesamtgewichts von Antisensibilitätsmittel zu Abscheidungsmittel 5:1 bis 30:1 beträgt.

13. Mundpflegezusammensetzung nach irgendeinem vorhergehenden Anspruch, bei der die Gesamtmenge an Antisensibilitätsmitteln 5 bis 15 Gew.-% der gesamten Zusammensetzung beträgt.

14. Mundpflegezusammensetzung nach irgendeinem vorhergehenden Anspruch, bei der die Zusammensetzung ferner Gellangummi umfasst.

15. Mundpflegezusammensetzung nach irgendeinem vorhergehenden Anspruch zur Verwendung bei der Behandlung von Zahnüberempfindlichkeit.

## Revendications

1. Composition pour le soin oral comprenant un agent modérant la sensibilité comprenant un mélange de silice hydratée, d'un citrate de potassium, et d'hydroxyapatite de calcium et d'un agent d'aide au dépôt polymère comprenant un polymère à base d'un copolymère de méthylvinyléther et d'anhydride maléique et un agent épaississant polymère comprenant de la gomme xanthane, la composition ayant une viscosité 3 semaines ou plus après fabrication de 10 000 à 60 000 cP à 25°C (mesurée en utilisant un viscosimètre Brookfield avec un support Helipath et une barre en T, broche TB, tournant à 5 tr/min).

2. Composition pour le soin oral selon la revendication 1, dans laquelle la composition présentant une viscosité 3 semaines ou plus après fabrication de 15 000 à 45 000 cP à 25°C.

3. Composition pour le soin oral selon la revendication 1 ou revendication 2, dans laquelle l'agent de dépôt polymère comprend un polymère et/ou copolymère soluble dans l'eau ayant des groupes latéraux anioniques le long de la chaîne principale polymère.

4. Composition pour le soin oral selon l'une quelconque des revendications précédentes, dans laquelle la teneur en agent de dépôt est de 0,005 à 4 % en masse de la composition totale.

5. Composition pour le soin oral selon l'une quelconque des revendications précédentes, dans laquelle la silice hydratée présente une taille moyenne de particule de 1 à 6 µm.

6. Composition pour le soin oral selon l'une quelconque des revendications précédentes, dans laquelle la teneur en silice hydratée dans la composition totale est de 1 à 10 % en masse de la composition totale.

7. Composition pour le soin oral selon l'une quelconque des revendications précédentes, dans laquelle la teneur en citrate de potassium est de 1 à 10 % en masse de la composition totale.

8. Composition pour le soin oral selon l'une quelconque des revendications précédentes, dans laquelle la teneur en hydroxyapatite de calcium est de 1 à 8 % en masse de la composition totale.

9. Composition pour le soin oral selon l'une quelconque des revendications précédentes, dans laquelle le rapport en masse d'hydroxyapatite de calcium à silice hydratée est de 1:1 à 1:4.

10. Composition pour le soin oral selon l'une quelconque des revendications précédentes, dans laquelle le rapport en masse de citrate de potassium à silice hydratée est de 1:1 à 1:4.

11. Composition pour le soin oral selon l'une quelconque des revendications précédentes, dans laquelle le rapport en masse de citrate de potassium à hydroxyapatite de calcium est de 2:1 à 1:2.

12. Composition pour le soin oral selon l'une quelconque des revendications précédentes, dans laquelle le rapport en masse de la masse totale d'agent anti-sensibilité à agent de dépôt est de 5:1 à 30:1.

13. Composition pour le soin oral selon l'une quelconque des revendications précédentes, dans laquelle la teneur totale en agents anti-sensibilité est de 5 à 15 % en masse de la composition totale.

14. Composition pour le soin oral selon l'une quelconque des revendications précédentes, dans laquelle la composition comprend de plus de la gomme gellane.

15. Composition pour le soin oral selon l'une quelconque des revendications précédentes pour une utilisation dans le traitement de l'hypersensibilité dentaire.
